Europäisches Patentamt

European Patent Office

Office européen des brevets

(11) Veröffentlichungsnummer: **0 348 765**

**A1**

(12) **EUROPÄISCHE PATENTANMELDUNG**

(21) Anmeldenummer: 89111038.9

(22) Anmeldetag: 19.06.89

(51) Int. Cl.⁴: **A61N 1/30**

(30) Priorität: 25.06.88 DE 3821519

(43) Veröffentlichungstag der Anmeldung:
03.01.90 Patentblatt 90/01

(84) Benannte Vertragsstaaten:
**AT BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Ruebsamen, Klaus, Dr.**
**Kaiserstuhl 6**
**D-6730 Neustadt(DE)**
Erfinder: **Kolter, Karl, Dr.**
**Sachsenweg 12**
**D-6703 Limburgerhof(DE)**
Erfinder: **Gruenhagen, Hans-Heinrich, Dr.**
**In den Weihergaerten 59**
**D-6700 Ludwigshafen(DE)**
Erfinder: **Naarmann, Herbert, Dr.**
**Haardtblick 15**
**D-6719 Wattenheim(DE)**
Erfinder: **Freyschlag, Herwig, Dr.**
**4. Gartenweg 9 b**
**D-6700 Ludwigshafen(DE)**
Erfinder: **Koenig, Horst, Prof.Dr.**
**Pariser Strasse 4**
**D-6700 Ludwigshafen(DE)**

(54) **Wirkstoffpflaster.**

(57) Es wird ein Wirkstoffpflaster beschrieben, welches aus einer elektrisch leitfähigen Schicht aus Kunststoff, einem Anschlußstück zur Verbindung mit einer Stromquelle und einer mit Wirkstoff getränkten Schicht besteht.

EP 0 348 765 A1

## Wirkstoffpflaster

Die vorliegende Erfindung betrifft eine flexible dermale Applikationsform für Wirkstoffe.

Die transdermale Applikation von ionisierten pharmakologischen Wirkstoffen mit Hilfe des elektrischen Stromes durch die Haut ist bereits bekannt, vgl. Pharmaceutical Research 3 (1986), 318.

Es ist bekannt, daß als Elektrodenmaterial Zinn, Kupfer, rostfreier Stahl oder ein starres Polymermaterial verwendet werden kann. Dieses wird mit einem 1 - 2 cm dicken Schwamm bedeckt. Nachteilig sind hierbei sowohl die relativ große, vom Schwamm aufgenommene Substanzmenge als auch die erforderlichen Manipulationen zur Befestigung der starren Elektroden auf der Hautoberfläche. Im ungünstigsten Fall können mit dieser Anordnung bei fehlender Distanz der Elektrode zur Haut Verbrennungen auftreten, die aufgrund der analgetischen Wirkung des elektrischen Stromes vom Patienten nicht oder erst spät wahrgenommen werden. Weiterhin können aus metallischen Elektroden Metallionen freigesetzt werden, welche bekanntermaßen zu Allergisierung führen können (Pharmaceutical Research 4, 1987, 438).

Es wurde nun gefunden, daß sich diese Nachteile vermeiden lassen, wenn man als Elektrodenmaterial elektrisch leitfähige, flexible Kunststoffe oder Kunststoffgewebe verwendet.

Gegenstand der Erfindung ist ein Wirkstoffpflaster, bestehend aus einer elektrisch leitfähigen Schicht mit einem Anschlußstück zur Verbindung mit der Stromquelle und einer mit Wirkstoff getränkten Schicht, dadurch gekennzeichnet, daß die elektrisch leitfähige Schicht aus einem flexiblen Kunststoff besteht.

Als Kunststoffe für die elektrisch leitfähige Schicht (auch "Polymerelektrode" genannt) kommen solche in Betracht, die sich flächig an Körperformen anpassen lassen und den Strom leiten. Solche Kunststoffe sind neben flexiblen, elektrisch leitfähigen Folien und Fasern insbesondere flexible Folien, z.B. auf Silikonbasis, sowie Vliese und Gewebe aus Polypropylen und andere Gewebe, die mit Polypyrrol oder analogen Heterozyklen beschichtet sind (US 4 468 291, DE 3 520 980, EP 206 133).

Aus diesen Kunststoffen werden geeignete Folien, Gewebe oder Vliese hergestellt, die bis 5 mm, vorzugsweise 10 μm - 3 000 μm dick sind. Das kann mit Hilfe von Walzen, Rakeln, Gieß- oder Sprüheinrichtungen bzw. Webe-oder Preßeinrichtungen geschehen.

Auf diesen Folien, Geweben oder Vliesen, d.h. auf der elektrisch leitfähigen Schicht, wird eine saugfähige Schicht befestigt, die den Wirkstoff aufnehmen kann. Das geschieht mit Hilfe von punktuell aufgebrachten Klebern auf der Basis von Polyacrylat, Polymethacrylat, Polyisobutylen, Silikonen, Naturkautschuk oder Kohlenwasserstoff-Harzen.

Als saugfähige Schichten eignen sich Schwämme und schwammartige Gewebe sowie Vliese. Beispiele hierfür sind Freudenberg-Vlies M 1556, mikroporöse Folien und makroporöse Folien.

Die Folie, das Gewebe oder Vlies aus leitfähigen Polymeren können auch selbst zur Aufnahme einer wirkstoffhaltigen Lösung bzw. eines Gels dienen. Als Monomere eignen sich zur Herstellung der leitfähigen Polymere vorzugsweise 5-Ring-Heterocyclen wie Pyrrol und dessen Derivate. Solche Derivate sind insbesondere in 3- und/oder 4-Stellung durch $C_{1-12}$-Alkylgruppen, vorzugsweise Methyl-, Ethyl- oder Propylgruppen, sowie durch Cl-, CN-, COO-und $SO_3H$-Gruppen substituierte 5-Ringe. Als 5-Ring ist neben Pyrrol auch beispielsweise Thiophen geeignet. Anstelle der 5-Ringe können auch oligomere 5-Ring-Heterocyclen wie Dipyrrol, Dithienyl und Trithienyl verwendet werden.

Die Folien können zur Anpassung an Körperformen, z.B. als Fingerlinge, Gelenkbandagen oder Strümpfe ausgebildet sein.

Die noch freie Seite der Folie, d.h. der Polymerelektrode, wird auf eine mit Klebstoff beschichtete zweite Folie geklebt, so daß ein Kleberand übersteht. Alternativ dazu kann die der Haut zugewandte Seite klebend ausgebildet sein.

Die Wirkstoffe werden in Wasser, Puffer oder Gel eingearbeitet. Mit dieser Wirkstoffzubereitung wird die saugfähige Schicht getränkt. Die freie Seite der saugfähigen Schicht wird mit Hilfe einer abziehbaren Schutzfolie abgedeckt. Als abziehbare Schutzfolie kommen beispielsweise silikonisierte Aluminiumfolien, silikonisierte PVC-, PE-, Polyamid und Polyesterfolien in Betracht.

Alternativ kann die Wirkstoffdotierung durch Herstellung des Polymers in Gegenwart des Wirkstoffs erfolgen. Neben konventionellen Stromquellen (z.B. Netzgeräte oder Batterien) kann das Wirkstoffpflaster mit einer ebenfalls flexibel gestalteten Batterie auf Basis leitfähiger Polymerer (vgl. "BASF Kunststoffe, Forschung und Entwicklung, herausgegeben von der BASF Aktiengesellschaft, 1986 Seite 40) betrieben werden.

Als Wirkstoffe eignen sich solche, die transdermal angewendet werden können. Obwohl die Wirkstoffe mit Hilfe von Strom durch die Haut transportiert werden, können als solche nicht nur ionische Wirkstoffe verwendet werden, sondern überraschenderweise auch nicht-ionische.

Folgende Wirkstoffgruppen sind insbesondere zu nennen: β-Blocker, Vasodilatatoren, Antihypertensiva, Dermatika, Steroide, Antirheumatika, Analgetika, Calciumantagonisten, Antiarrhythmika, Psychopharmaka, Antiparkinsonmittel, Antikoagulantia, Anticholinergika, Antihistaminika, Antiphlogistika, Hormone, Peptide und Proteine. Im einzelnen seien vorzugsweise folgende Wirkstoffe genannt. Propranolol, Metoprolol, Soquinolol, Clonidin, Dexamethason, Hydrocortison, Diclofenac, Indomethacin, Bufexamac, Phenylbutazon, Hydromorphon, Oxycodon, Morphin, Fentanyl, Verapamil, Gallopamil, Nifedipin, Diltiazem, Propafenon, Haloperidol, Biperiden, Atropin, Diphenhydramin, Testosteron, Progesteron, Östradiol, Heparin, TRH, Vasopressin, Insulin und Tumornekrosefaktor.

Beispiele zur Gestaltung von Polymerelektroden für unterschiedliche Anwendungsbereiche

1. Diclofenac-Iontophorese-Pflaster

Ein textilähnliches Gewebe auf der Basis von Polypropylen wurde 1 min mit einer Lösung von 5% Pyrrol und 5% Benzolsulfonsäure in Ethanol (Lösung I) getränkt und danach mit 5%iger wäßriger Lösung von Natriumperoxodisulfat ($Na_2S_2O_8$) (Lösung II) versetzt. Nach 10 min Kontamination wurde das Gewebe aus der Lösung herausgenommen, mit Wasser gespült und 5 h bei 50°C getrocknet. Die Leitfähigkeit des so erhaltenen Gewebes lag bei $2 \cdot 10^{-3}$ Siemens/cm.

Aus diesem Gewebe wurde eine rechteckige, 10 x 15 cm große Fläche ausgestanzt, auf eine mit Polybutylacrylatkleber versehene Polyester-Folie (12 x 17 cm) aufgebracht und mit Hilfe einer Quetschverbindung mit einem flexiblen Kabel leitend verbunden. 100 mg Wirkstoff wurden in Form eines 0,5%igen Hydroxypropylmethylzellulosegels auf ein mittels punktförmiger Klebestellen mit der Elektrode verbundenes, 1,5 mm dickes Freudenberg-Vlies (M 1556) aufgetragen, so daß sich das Vlies mit dem Gel homogen durchtränkte und eine leitende Verbindung zur Elektrode einging. Die zur Haut gerichtete Seite samt überstehender Klebefolie wurde durch eine silikonisierte, 50 μm dicke Aluminiumfolie abgedeckt, die vor der Applikation des Pflasters abgezogen wird.

2. Iontophorese-Bandage zur Behandlung von Thrombophlebitis

Ein 2 mm dickes, 10 x 30 cm großes, hochflexibles, vliesähnliches Gewebe aus Polypyrrolfasern wurde mit 320 ml Lösung I und 240 ml Lösung II analog Beispiel 1 behandelt. Nach Weiterbearbeitung wie in Beispiel 1 erhielt man ein Gewebe mit einem leitfähigen, fest haftenden Überzug der Leitfähigkeit $3,5 \cdot 10^{-2}$ Siemens/cm. Dieses wurde durch eine Quetschverbindung mit einem flexiblen Kabel verbunden. Die Rückseite des Elektrodengewebes wurde durch eine Polyamidfolie flüssigkeitsdicht versiegelt. Die Elektrode wurde mit einer 0,25%igen Heparinlösung mit 1% Hydroxypropylmethylzellulose als Gelbildner getränkt. Die zur Haut gerichtete Seite der Elektrode wurde durch eine mikroporöse Folie (®Celgard 3401, Celanese) verschlossen. Die Elektrode wurde in aufgerollter Form eingesiegelt konfektioniert. Zur Fixierung auf der Extremitätenoberfläche wurde die Bandage mit Klettenverschlüssen befestigt.

3. Heparin-Pflaster

A. Heparin-Folie

a) Einstufenverfahren

In eine Elektrolyse-Zelle mit Nickelelektroden (Größe 10x10 $cm^2$) und einem Abstand von 1,5 cm wurde ein Elektrolyt (1600 ml Wasser, 8 g Pyrrol, 5 g Heparin-Natrium) gefüllt. Bei einer Temperatur von 22°C wurde 90 min mit einer Stromdichte [i] von 2 mA/$cm^2$ anodial polymerisiert. Auf der Anode entstand dabei ein schwarzer Film, der abgelöst wurde. Der Film besaß eine elektrische Leitfähigkeit von 15 Siemens/cm und zeigte folgende elementaranalytischen Kenndaten: C:61%; H:6,7%; N:10,9%; 0:16,3%; S:4,9%.

Der Film war freistehend 60 μm dick und enthielt den Wirkstoff in ionogener Form.

b) Zweistufenverfahren

In eine Elektrolyse-Zelle mit Platinelektroden (Größe 10x10 $cm^2$) und einem Abstand von 2 cm wurde ein Elektrolyt (1600 ml Wasser, 8 g Pyrrol, 1 g phenylsulfonsaures Natrium) gefüllt. Bei einer Temperatur von 26°C wurde 30 min mit einer Stromdichte [i] von 3 mA/$cm^2$ anodisch polymerisiert. Auf der Elektrode bildete sich ein Polymerfilm, der eine Dicke von 20 μm und eine Leitfähigkeit von 25 Siemens/cm besaß.

Dieser Film wurde von der Elektrode abgelöst und anstelle der Platinelektrode als selbständige Anode geschaltet.

Der Elektrolyt wurde gegen einen neuen (1000 ml Wasser, 5 g Pyrrol, 5 g Heparin-Natriumsalz) ausgetauscht. Nach einer Polymerisationszeit von 180 min bei 22°C und einer Stromdichte [i] von 1,5 mA/$cm^2$ entstand ein glatter einheitlicher Film mit einer Dicke von 75 μm und einer elektrischen Leitfähigkeit von 14 Siemens/cm.

Der Film war freistehend und flexibel, er enthielt den Wirkstoff in ionogener Form.

c) Pflasterherstellung

Die Folie wurde auf einer Polyester-Folie (12,5x12,5 cm²) befestigt und mit einem flexiblen Kabel leitend verbunden. Die zur Haut gerichtete Seite der elektrisch leitfähigen Schicht wurde mit einer Abziehfolie, die am Kleberand befestigt war, verschlossen.

B. Elastische Heparin-Folie

In eine Elektrolysezelle wurden 3 g Heparin-Natriumsalz, 10 g Pyrrol, 100 g Naturlatex (60% Feststoffgehalt) sowie 1000 ml Wasser gegeben. Die Elektroden (10x10 cm²) bestanden aus Platin und waren in einem Abstand von 2 cm angeordnet. Bei einer Stromdichte [i] von 10 mA/cm² und einer Zellspannung von 4,5 V war nach einer Elektrolysedauer von 3 h bei 20° C ein 70 μm dicker gummielastischer Film entstanden, der von der Platinanode abgezogen wurde. Der Film war schwarz und hatte eine elektrische Leitfähigkeit von 0,65 Siemens/cm. Er enthält das Heparin und kann bis auf 250 % verstreckt werden.

Ansprüche

1. Wirkstoffpflaster, bestehend aus einer elektrisch leitfähigen Schicht mit einem Anschlußstück zur Verbindung mit der Stromquelle und einer mit Wirkstoff getränkten Schicht, dadurch gekennzeichnet, daß die elektrisch leitfähige Schicht aus einem flexiblen Kunststoff besteht.

2. Wirkstoffpflaster gemäß Anspruch 1, dadurch gekennzeichnet, daß die mit Wirkstoff getränkte Schicht ein Teil der elektrisch leitfähigen Schicht ist.

3. Wirkstoffpflaster gemäß Anspruch 1, dadurch gekennzeichnet, daß der Wirkstoff ionisiert oder teilweise ionisiert vorliegt.

4. Wirkstoffpflaster gemäß Anspruch 1, dadurch gekennzeichnet, daß der Wirkstoff nicht ionisiert ist.

5. Wirkstoffpflaster gemäß Anspruch 1, dadurch gekennzeichnet, daß die Stromquelle aus einer flexiblen primären oder sekundären Batterie auf der Basis leitfähiger Polymerer besteht.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 240 593 (DRUG DELIVERY SYSTEMS INC.)<br>* Seite 3, Zeilen 1-52; Seite 5, Zeile 53 - Seite 6, Zeile 35 *<br>--- | 1-3,5 | A 61 N 1/30 |
| X | EP-A-0 138 347 (SASAKI)<br>* Seite 18, Zeile 28 - Seite 21, Zeile 2 *<br>--- | 1-3 | |
| X | EP-A-0 269 246 (KONNO)<br>* Seite 4, Zeile 39 - Seite 5, Zeile 3 *<br>----- | 1,4 | |

RECHERCHIERTE SACHGEBIETE (Int. Cl.4)

A 61 N

Der vorliegende Recherchenbericht wurde für alle Patentansprüche erstellt

| Recherchenort | Abschlußdatum der Recherche | Prüfer |
|---|---|---|
| DEN HAAG | 28-09-1989 | LEMERCIER D.L.L. |